# EUROPEAN PATENT APPLICATION

(11) **EP 4 717 251 A2**
(43) Date of publication of application: **01.04.2026**
(21) Application number: 25203230.5
(22) Date of filing: 19.09.2025
(51) Int. Cl.: A61K 6/818, A61K 6/836, A61K 6/887, A61K 6/17, A61K 6/64, A61K 6/71, A61K 6/76

(54) **DENTAL CURABLE COMPOSITION HAVING HIGH MECHANICAL STRENGTH**

(30) Priority: 28.09.2024 JP 2024169693
(71) Applicant: Shofu Inc., Kyoto-shi, Kyoto 605-0983 (JP)
(72) Inventor: TAKAHASHI, Shuhei, Kyoto-shi, Kyoto, 605-0983 (JP); OBATA, Atsushi, Kyoto-shi, Kyoto, 605-0983 (JP); KAWAI, Yutaka, Kyoto-shi, Kyoto, 605-0983 (JP)
(74) Representative: Eisenführ Speiser

(57) **Abstract**

To provide a dental curable composition that can achieve high mechanical strength after curing.

To provide a dental curable composition comprising a silane coupling material represented by formula (1) and/or an inorganic filler surface-treated with silane coupling material represented by formula (1).

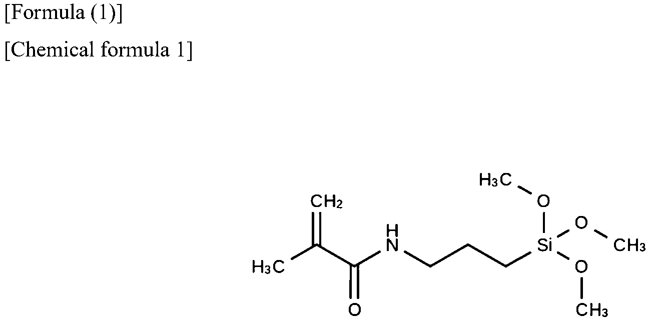

## Description

### BACKGROUND OF THE INVENTION

### Field of the Invention

The present invention relates to a dental curable composition containing a radical polymerizable coupling agent having a methacrylamide structure.

### Description of the Related Art

In dentistry, dental curable compositions consisting of a polymerizable monomer, an inorganic filler and a polymerization initiator have been widely used. These are generally called a dental composite resin and utilized for various uses such as direct restorative materials for tooth defective parts due to dental caries, etc., dental crown prosthesis restorative materials such as inlays, crowns, and bridges, abutment tooth building materials and dental block materials for CAD/CAM.

Generally, in a dental composite resin, a (meth)acrylic acid derivative monomer such as methyl methacrylate, triethylene glycol dimethacrylate and urethane dimethacrylate has been used. In the free radical polymerization (hereinafter referred to as radical polymerization) of vinyl monomers such as these (meth)acrylic acid derivative monomers, a high molecular weight substance is formed to cure by cleaving the carbon-carbon double bond to form a single bond. In addition to vinyl monomers, inorganic fillers are added to composite resins to improve their mechanical strength. Generally, these inorganic fillers are surface-treated with silane coupling material having a polymerizable group to improve wettability and mechanical strength.

In the dental field, γ-methacryloxypropy ltrimethoxysilane (hereinafter referred to as KBM-503) has been widely used as a silane coupling material. When an inorganic filler is surface-treated with this compound, there is a problem that because hydrophobicity is low, hydrolysis easily progresses and therefore material durability is slow. Therefore, there is a drawback that sufficient mechanical strength is not achieved.

Therefore, in order to improve the durability and filling rate of the material, various methods have been proposed, including a method using a radical polymerizable silane coupling material having a cyclic compound as a skeleton (Japanease Patent No. 7118549), a method using a silane coupling material having many polymerizable groups (Japanese Patent No. 5416447), a method using a silane coupling material with a long alkyl chain (Japanease Patent No. 6220723), a method using a silane coupling material having a hydroxyl group and a urethane group (Japanease Patent No. 7104490), a method using a silane coupling material having an epoxy ring and a urethane group (Japanese Patent No. 7104489), a method using a silane coupling material having a fluorine-containing organic group (Japanease Patent No. 7093628), a method of using two types of silane coupling materials in combination (Japanese Patent No. 6220723 and (Japanese Patent No.6793241), a method using a radical polymerizable silane coupling material having a urethane bond (Japanese Patent No. 6841915, Japanese Patent No. 7104488, Japanese Patent No. 7104487 and Japanese Patent No. 7104486).

### SUMMARY OF THE INVENTION

### Technical Problem

However, when the methods described in the above documents were applied to dental resin cured body and the like, there has been a room for improvement in mechanical strength.

An object of the present invention is to provide a dental curable composition that can achieve high mechanical strength after curing.

### Solution to Problem

As a result of the intensive study by the present inventors, it has been found that, by surface-treating an inorganic filler using a silane coupling material represented by a chemical structure formula (1) described below, it is possible to impart high affinity with respect to a radical polymerizable monomer, particularly a radical polymerizable monomer containing an urethane bond or a hydrogen bond and high dispersibility of an inorganic filler. This makes it possible to impart high mechanical strength in the case of using in the dental curable composition. The present invention is based on the above findings.

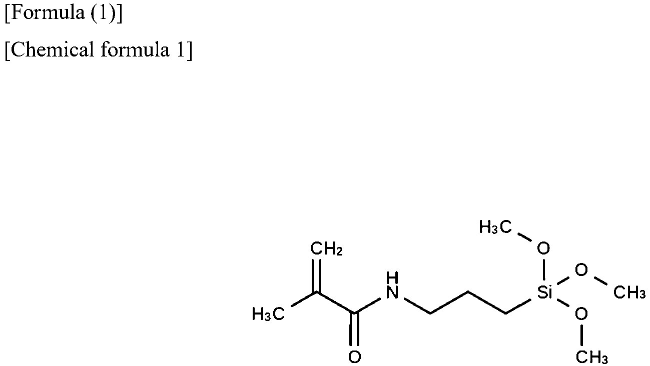

The present invention provides a dental curable composition comprising a silane coupling material represented by formula (1) and/or an inorganic filler surface-treated with silane coupling material represented by formula (1).

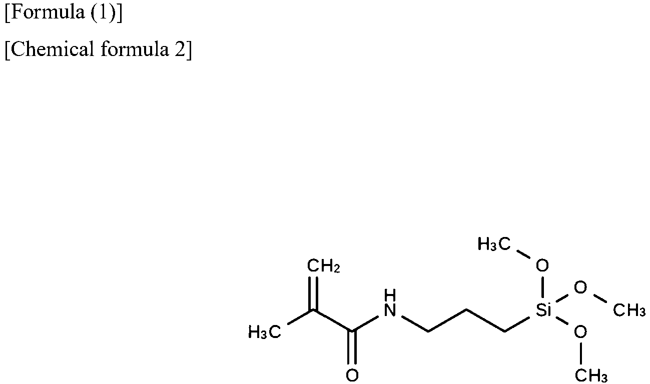

### Advantageous Effects of Invention

According to the dental curable composition of the present invention, it is possible to achieve high mechanical strength after curing.

### DETAILED DESCRIPTION OF THE PREFERRED EMBODIMENTS

The dental curable composition of the present invention comprises a silane coupling material represented by formula (1) and/or an inorganic filler surface-treated with silane coupling material represented by formula (1).

By surface treatment of an inorganic filler with a silane coupling material represented by formula (1), high affinity with respect to a radical polymerizable monomer, particularly a radical polymerizable monomer containing an urethane bond and high dispersibility of an inorganic filler are exhibited to impart high mechanical strength and flexibility to the cured body of the dental curable composition.

This high affinity effect is particularly exhibited in the case that the radical polymerizable monomer has an urethane bond or a hydrogen bond. This is considered to be due to the presence of a nitrogen atom and an amide bond within formula (1).

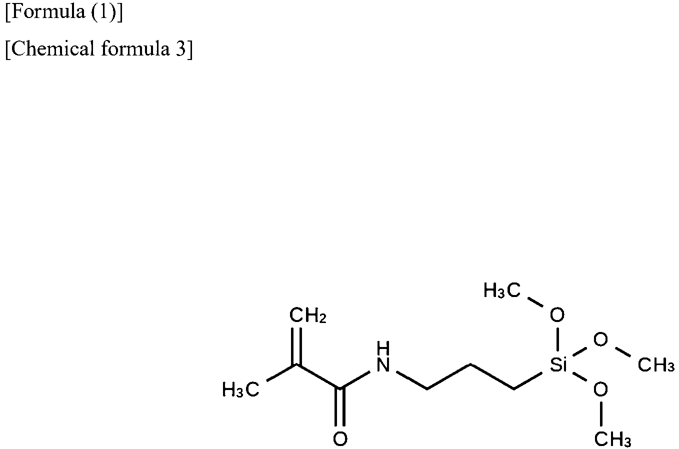

It is considered that in the inorganic filler surface-treated with silane coupling material represented by Formula (1), a nitrogen atom and an amide bond are introduced onto its surface to exhibit high affinity for a radical polymerizable monomer having an urethane bond or a hydrogen bond.

Further, it is also considered that the presence of an amide bond forms hydrogen bond between amide groups to strength a bond between inorganic fillers surface-treated with silane coupling material. In the present invention, it is possible to highly fill an inorganic filler and as a result, it is possible to achieve high mechanical strength.

The dental curable composition of the present invention may comprise the inorganic filler surface-treated with silane coupling material represented by formula (1).

In the dental curable composition of the present invention, a treatment amount of the silane coupling material in the surface treatment may be 0.1 to 20 parts by weight with respect to 100 parts by weight of the inorganic filler.

In the dental curable composition of the present invention, a compounding amount of the inorganic filler surface-treated with silane coupling material represented by formula (1) may be 1 to 90 parts by weight with respect to 100 parts by weight of the whole of the dental curable composition.

The dental curable composition of the present invention may further comprise a radical polymerizable monomer, and a polymerization initiator.

The dental curable composition of the present invention may further comprise an inorganic filler surface-treated with a surface treatment agent other than the silane coupling material represented by formula (1).

In the dental curable composition of the present invention, the radical polymerizable monomer may have a urethane bond.

In the dental curable composition of the present invention, the radical polymerizable monomer may have one or more hydroxyl groups and two or more polymerizable functional groups.

In the dental curable composition of the present invention, the radical polymerizable monomer may include a monomer represented by formula (2).

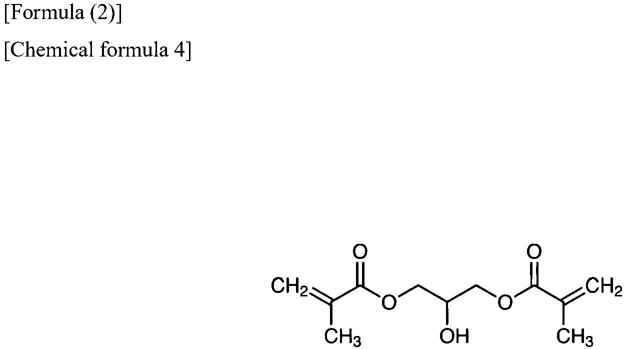

In the dental curable composition of the present invention, a compounding amount of the radical polymerizable monomer containing the compound represented by formula (2) may be 1 to 10 parts by weight with respect to 100 parts by weight of the whole of the dental curable composition.

In the dental curable composition of the present invention, a weight ratio of the silane coupling material represented by formula (1) to the radical polymerizable monomer including the compound represented by formula (2) may be in a range of 1:0.5 to 1:20.

In the dental curable composition of the present invention, the inorganic filler surface-treated with silane coupling material represented by formula (1) may be a spherical nanofiller having an average particle diameter of 0.01 to 1 µm.

In the dental curable composition of the present invention, the inorganic filler surface-treated with silane coupling material represented by formula (1) may be a crushed type inorganic filler having an average particle diameter of 0.01 to 10 µm.

In the dental curable composition of the present invention, the inorganic filler surface-treated with silane coupling material represented by formula (1) may be an aggregated type inorganic filler having an average particle diameter of 0.01 to 10 µm.

In the dental curable composition of the present invention, the aggregated type inorganic filler may consist of SiO₂: 50-99 % by weight and ZrO₂: 1-50 % by weight.

In the dental curable composition of the present invention, a ten hour half-life temperature of the polymerization initiator may be between 70 °C and 170 °C.

In the dental curable composition of the present invention, a cured body of the dental curable composition may have a bending strength of 300 MPa or more after storage in water at 37 °C for one week.

In the dental curable composition of the present invention, a cured body of the dental curable composition may have a flexural modulus of 20 GPa or more after storage in water at 37 °C for one week.

The dental curable composition of the present invention may be used in manufacture of a dental resin cured body for cutting.

The dental curable composition of the present invention may be used in manufacture of a dental composite resin.

The dental curable composition of the present invention may be used as a dental curable composition for 3D printer.

The present invention provides an inorganic filler surface-treated with silane coupling material represented by formula (1).

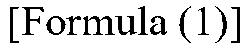

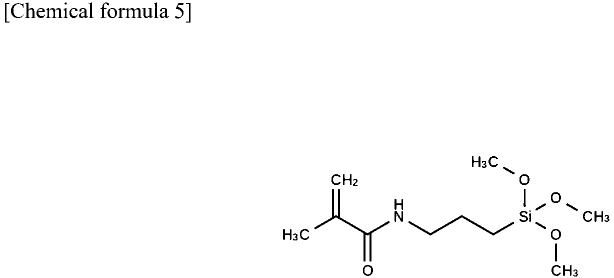

### [Silane coupling material represented by formula (1)]

The silane coupling material used in the present invention has a methacrylamide structure, specifically the structure shown in formula (1). In the present invention, the silane coupling material represented by formula (1) may be used alone or in combination with other surface treatment agent for surface treatment. Examples of other surface treatment agent include, but not limited to, 3-methacryloxypropyl trimethoxysilane, 8-methacryloxyoctyl trimethoxysilane, titanium acetylacetonate and zirconium ethyl acetoacetate.

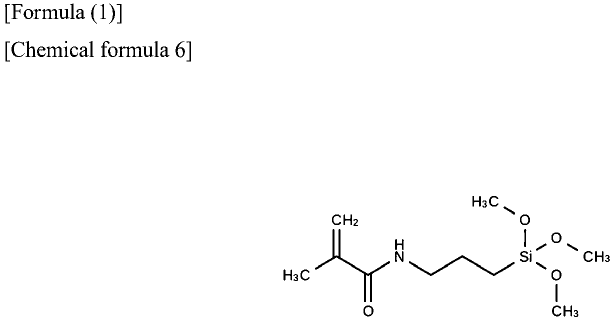

The dental curable composition of the present invention may contain the silane coupling material represented by formula (1) in a form of pre-surface-treatment, that is, as the silane coupling material represented by formula (1) itself, or in a form of post-surface-treatment, that is, as an inorganic filler surface-treated with silane coupling material represented by formula (1). The dental curable composition of the present invention may contain both the silane coupling material represented by formula (1) itself and the inorganic filler surface-treated with silane coupling material represented by formula (1). The dental curable composition of the present invention may not contain the silane coupling material represented by formula (1) itself and contain the inorganic filler surface-treated with silane coupling material represented by formula (1). The dental curable composition of the present invention may not contain the inorganic filler surface-treated with silane coupling material represented by formula (1) and contain the silane coupling material represented by formula (1) itself.

When the dental curable composition contains the silane coupling material represented by formula (1) itself in the present invention, the composition ratio of the silane coupling material represented by formula (1) may be 0.05 to 5.0 % by weight of the whole of the dental curable composition in the case that the dental curable composition does not contain a polymerization initiator, and may be 0.05 to 5.0 % by weight of the dental curable composition excluding a polymerization initiator in the case that the dental curable composition contains a polymerization initiator. In other words, when the dental curable composition does not contain a polymerization initiator, the composition ratio may be 0.05 to 5.0 parts by weight with respect to 100 parts by weight of the whole of the dental curable composition. When the dental curable composition contains a polymerization initiator, the composition ratio may be 0.05 to 5.0 parts by weight with respect to 100 parts by weight of the dental curable composition excluding the polymerization initiator. When the content is lower than 0.05 % by weight, there is a case where the silane coupling material is not introduced sufficiently. Furthermore, when the content exceeds 5.0 % by weight, there is a case where a condensation product consisting solely of the silane coupling material may form, and the mechanical strength of the cured product is affected.

When the dental curable composition contains the inorganic filler surface-treated with silane coupling material represented by formula (1) in the present invention, the treatment concentration in the surface treatment of the inorganic filler using a silane coupling material represented by formula (1) may be 0.1 to 20 % by weight of the inorganic filler. In other words, the treatment amount of a silane coupling material with respect to 100 parts by weight of the inorganic filler in the surface treatment may be 0.1 to 20 parts by weight. When the treatment amount is lower than 0.1 % by weight, there is a case where the silane coupling material is not introduced sufficiently. Furthermore, when the treatment amount exceeds 20% by weight, there is a case where a condensation product consisting solely of the silane coupling material may form, and the mechanical strength of the cured product is affected.

### [Inorganic filler]

The inorganic filler that can be used in the present invention is not particularly limited, but Examples include silicon dioxide, alumina, silica-titania, silica-zirconia, silica-alumina, borosilicate glass, soda glass, barium glass, strontium glass, glass ceramic, aluminosilicate glass, barium borosilicate glass, strontium borosilicate glass, fluorosilicate glass, and strontium calcium fluorosilicate glass. The dental curable composition of the present invention may contain these inorganic fillers in a state after surface treatment with a silane coupling material represented by formula (1). The dental curable composition of the present invention may contain these inorganic fillers in a state before surface treatment with a silane coupling material represented by formula (1). When the dental curable composition of the present invention contains the silane coupling material represented by formula (1) itself, it is possible to contain only the inorganic filler that have not been surface-treated with silane coupling material represented by formula (1) as the inorganic filler.

When the dental curable composition contains the silane coupling material represented by formula (1) itself in the present invention, the composition ratio of the inorganic filler before surface treatment with silane coupling material represented by formula (1) in the dental curable composition of the present invention is not particularly limited. However, when the dental curable composition does not contain a polymerization initiator, the composition ratio may be 0.95 to 85 % by weight of the whole of the dental curable composition, and may be 50 to 85 % by weight. When the dental curable composition contains a polymerization initiator, the composition ratio may be 0.95 to 85 % by weight of the dental curable composition excluding the polymerization initiator and may be 50 to 85 % by weight. That is, when the dental curable composition does not contain a polymerization initiator, the composition ratio may be 0.95 to 85 parts by weight with respect to 100 parts by weight of the whole of the dental curable composition, and may be 50 to 85 parts by weight. When the dental curable composition contains a polymerization initiator, the composition ratio may be 0.95 to 85 parts by weight with respect to 100 parts by weight of the dental curable composition excluding the polymerization initiator, and may be 50 to 85 parts by weight. When the content is lower than 0.95 % by weight, there is a case where the mechanical strength of the cured product decreases. Furthermore, when the content exceeds 85 % by weight, there is a case where the viscosity of the prepared paste is too high to decrease operability.

The composition ratio of the inorganic filler after surface-treatment with the silane coupling material represented by formula (1) in the dental curable composition of the present invention is not particularly limited. However, when the dental curable composition does not contain a polymerization initiator, the composition ratio may be 1 to 90 % by weight of the whole of the dental curable composition, and may be 70 to 85 % by weight. When the dental curable composition contains a polymerization initiator, the composition ratio may be 1 to 90 % by weight of the dental curable composition excluding the polymerization initiator and may be 70 to 85 % by weight. That is, when the dental curable composition does not contain a polymerization initiator, the composition ratio may be 1 to 90 parts by weight with respect to 100 parts by weight of the whole of the dental curable composition, and may be 70 to 85 parts by weight. When the dental curable composition contains a polymerization initiator, the composition ratio may be 1 to 90 parts by weight with respect to 100 parts by weight of the dental curable composition excluding the polymerization initiator, and may be 70 to 85 parts by weight. When the content is lower than 1 % by weight, there is a case where the mechanical strength of the cured product decreases. Furthermore, when the content exceeds 90 % by weight, there is a case where the viscosity of the prepared paste is high to decrease operability. When the dental curable composition of the present invention contains both the silane coupling material represented by formula (1) itself and the inorganic filler surface-treated with silane coupling material represented by formula (1), the composition ratio after surface treatment with the silane coupling material represented by formula (1) means the composition ratio after surface treatment with the silane coupling material represented by formula (1) itself contained in the dental curable composition. That is, it means the composition ratio of the total of a surface-treated product such as an inorganic filler that is surface-treated with silane coupling material represented by formula (1) itself and a surface-treated product such as an inorganic filler that has been surface-treated with the silane coupling material represented by formula (1) before mixing with the silane coupling material represented by formula (1) itself.

Examples of the shapes of the inorganic filler used in the present invention include spherical and irregular. The spherical inorganic filler means an inorganic filler having a rounded shape or an inorganic filler not having an arbitrary corner in the case of observing in a photograph of the inorganic filler taken with an electron microscope. By having the spherical shape, it is expected to exhibit a sliding effect and an effect of mitigate interference between fillers. The irregular inorganic filler means an irregular particle obtained by mechanical grinding. The irregular particle may have a shape having an arbitrary corner. By having the irregular shape, it is expected to exhibit an effect of suppressing fluidity and maintaining the shapability of paste. In the dental curable composition of the present invention, it is possible o use the spherical filler and the irregular filler in combination from the viewpoint of achieving both flowability and shapability of paste. Furthermore, it is possible to use two or more kinds of fillers having different shapes and/or different average particle diameters by mixing or combining. By combining two or more kinds of fillers, fillers are densely filled and the number of interaction points between the filler and the polymerizable monomer or between the fillers themselves increases.

The average particle diameter of the inorganic filler used in the present invention may be 0.01 to 100 µm, may be 0.01 to 20 µm, and may be 0.01 to 10 µm. Furthermore, in the present invention, the average particle diameter of the inorganic filler surface-treated with silane coupling material represented by formula (1) may be 0.01 to 100 µm, may be 0.01 to 20 µm, and may be 0.01 to 10 µm. When the average particle diameter of the inorganic filler is less than 0.01 µm, there is a case where stickiness occurs in the curable composition and the operability of the paste deteriorates. On the other hand, when the average particle diameter of the inorganic filler exceeds 100 µm, there is a case where the mechanical strength of the cured product decreases. By maintaining the average particle diameter of the inorganic filler within a range of 0.01 to 10 µm, it is possible to achieve appropriate paste dispersibility and to achieve high mechanical strength after curing. In the present invention, the average particle diameter of the inorganic filler may be measured, for example, using a particle size analyzer, a laser diffraction type particle size measuring device and an electron microscope. The average particle diameter of the inorganic filler is not substantially affected by surface treatment with a surface treatment agent. Therefore, the average particle diameter of the inorganic filler may be either in a state before surface treatment with a surface treatment agent or in a state after surface treatment with a surface treatment agent.

A shape of the inorganic filler is not particularly limited, may be a spherical nanofiller. By having a spherical shape, it is possible to exhibit excellent paste operability and to enhance a filling rate in the dental curable composition. A spherical nanofiller maeans a nano-scale filler having a rounded shape in the case of observing a photograph of the filler taken with an electron microscope. By having the spherical shape, it is expected to exhibit a sliding effect and an effect of mitigate interference between fillers. The average particle diameter of the spherical nanofiller may be 0.01 to 1 µm, may be 0.02 to 0.5 µm, and may be 0.05 to 0.1 µm. When the average particle diameter is less than 0.01 µm, there is a case where the specific surface area increases to increase the viscosity of the paste, and therefore the filler is not added sufficiently to decrease the mechanical strength of the cured product. On the other hand, when the average particle diameter exceeds 1 µm, there is a case where the surface area of the spherical filler decreases and therefore there is a case where it is not possible to obtain a cured product of the dental curable composition which has high shapability. The average particle diameter of the spherical nanofiller is not substantially affected by surface treatment with a surface treatment agent. Therefore, average particle diameter of the spherical nanofiller may be either in a state before surface treatment with a surface treatment agent or in a state after surface treatment with a surface treatment agent.

The spherical nanofiller used in the present invention may be a spherical nanofiller hydrophobic-treated with a surface treatment agent. The hydrophobic treatment may be performed before surface treatment with silane coupling material represented by formula (1) and may be performed after surface treatment with silane coupling material represented by formula (1). Furthermore, the spherical nanofiller may be not surface-treated with the silane coupling material represented by formula (1). By containing a hydrophobized spherical nanofiller, in addition to the sliding effect that occurs in the case of applying an external force, it is possible to exhibit an effect of improving the fluidity of the paste because the hydrophilic interaction occurring between the polymerizable monomer and the filler is weakened and the fluidity of the paste is not impaired in the case of applying an external force.

The dental curable composition of the present invention may contain a spherical nanofiller as the inorganic filler surface-treated with silane coupling material represented by formula (1). The composition ratio of the spherical nanofiller after surface-treatment with silane coupling material represented by formula (1) in the dental curable composition of the present invention is not particularly limited. However, when the dental curable composition does not contain a polymerization initiator, the composition ratio may be 1 to 15 % by weight of the whole of the dental curable composition, and may be 1 to 5 % by weight. When the dental curable composition contains a polymerization initiator, the composition ratio may be 1 to 15 % by weight of the dental curable composition excluding the polymerization initiator and may be 1 to 5 % by weight. That is, when the dental curable composition does not contain a polymerization initiator, the composition ratio may be 1 to 15 parts by weight with respect to 100 parts by weight of the whole of the dental curable composition, and may be 1 to 5 parts by weight. When the dental curable composition contains a polymerization initiator, the composition ratio may be 1 to 15 parts by weight with respect to 100 parts by weight of the dental curable composition excluding the polymerization initiator, and may be 1 to 5 parts by weight. When the content is lower than 1 % by weight, there is a case where the flowability of the paste decreases. Furthermore, when the content exceeds 15 % by weight, there is a case where the shapability of the paste decreases.

When the dental curable composition contains the silane coupling material represented by formula (1) itself in the present invention, the composition ratio of the spherical nanofiller before surface treatment with silane coupling material represented by formula (1) in the dental curable composition of the present invention is not particularly limited. However, when the dental curable composition does not contain a polymerization initiator, the composition ratio may be 1 to 14 % by weight of the whole of the dental curable composition, and may be 1 to 5 % by weight. When the dental curable composition contains a polymerization initiator, the composition ratio may be 1 to 14 % by weight of the dental curable composition excluding the polymerization initiator and may be 1 to 5 % by weight. That is, when the dental curable composition does not contain a polymerization initiator, the composition ratio may be 1 to 14 parts by weight with respect to 100 parts by weight of the whole of the dental curable composition, and may be 1 to 5 parts by weight. When the dental curable composition contains a polymerization initiator, the composition ratio may be 1 to 14 parts by weight with respect to 100 parts by weight of the dental curable composition excluding the polymerization initiator, and may be 1 to 5 parts by weight. When the content is lower than 1 % by weight, there is a case where the flowability of the paste decreases. Furthermore, when the content exceeds 14 % by weight, there is a case where the shapability of the paste decreases.

It is not necessary that the spherical nanofiller is manufactured or obtained as a single particle, and the spherical nanofiller may be obtained by mixing two or more spherical nanofillers with different average particle diameters and/or composition ratios, as long as the average particle diameter and/or composition ratio of each falls within the above ranges.

It is also effective to use a spherical nanofiller surface-treated with both the silane coupling material represented by formula (1) and a surface treatment agent other than the silane coupling material represented by formula (1).

The dental curable composition of the present invention may contain, in addition to the inorganic filler surface-treated with silane coupling material represented by formula (1), a spherical nanofiller not surface-treated with silane coupling material represented by formula (1) as other filler described below. When the dental curable composition of the present invention does not contain the silane coupling material represented by formula (1) itself, the dental curable composition of the present invention may contain, in addition to the inorganic filler surface-treated with silane coupling material represented by formula (1), a spherical nanofiller not surface-treated with silane coupling material represented by formula (1) as other filler described below.

The shape of the inorganic filler is not particularly limited, but may be a crushed type inorganic filler. When the inorganic filler is a crushed type inorganic filler, it is possible to impart shapability to the paste. The crushed type inorganic filler is an inorganic filler mechanically crushed and may have a shape with an arbitrary corner.

The average particle diameter of the crushed type inorganic filler used in the present invention may be 0.01 to 100 µm, and from the viewpoint of mechanical strength, may be 0.01 to 20 µm, or 0.01 to 10 µm. When the average particle diameter is less than 0.01 µm, there is a case where the specific surface area of the crushed inorganic filler increases and the flowability of the paste decreases. On the other hand, when the average particle diameter exceeds 100 µm, the dispersibility of the crushed type inorganic filler in the dental curable composition may deteriorate and it is impossible to manufacture a uniform paste.

The crushed type inorganic filler used in the present invention may be a crushed type inorganic filler hydrophobic-treated with a surface treatment agent. The hydrophobic treatment may be performed before surface treatment with silane coupling material represented by formula (1) and may be performed after surface treatment with silane coupling material represented by formula (1). Furthermore, the hydrophobic-treated crushed type inorganic filler may be not surface-treated with the silane coupling material represented by formula (1). By containing a hydrophobic-treated crushed type inorganic filler, it is possible to enhance shapability and improve the mechanical strength of the dental curable composition after curing.

The dental curable composition of the present invention may contain a crushed type inorganic filler as the inorganic filler surface-treated with silane coupling material represented by formula (1). The composition ratio of the crushed type inorganic filler after surface-treatment with silane coupling material represented by formula (1) in the dental curable composition of the present invention is not particularly limited. However, when the dental curable composition does not contain a polymerization initiator, the composition ratio may be 10 to 60 % by weight of the whole of the dental curable composition, and may be 20 to 50 % by weight. When the dental curable composition contains a polymerization initiator, the composition ratio may be 10 to 60 % by weight of the dental curable composition excluding the polymerization initiator and may be 20 to 50 % by weight. That is, when the dental curable composition does not contain a polymerization initiator, the composition ratio may be 10 to 60 parts by weight with respect to 100 parts by weight of the whole of the dental curable composition, and may be 20 to 50 parts by weight. When the dental curable composition contains a polymerization initiator, the composition ratio may be 10 to 60 parts by weight with respect to 100 parts by weight of the dental curable composition excluding the polymerization initiator, and may be 20 to 50 parts by weight. When the content is lower than 10 % by weight, there is a case where the mechanical strength of the cured product decreases. Furthermore, when the content exceeds 60 % by weight, the viscosity of the prepared paste is too high to decrease operability.

When the dental curable composition contains the silane coupling material represented by formula (1) itself in the present invention, the composition ratio of the crushed type inorganic filler that is not surface treated with the silane coupling material represented by formula (1) in the dental curable composition of the present invention is not particularly limited. However, when the dental curable composition does not contain a polymerization initiator, the composition ratio may be 10 to 50 % by weight of the whole of the dental curable composition, and may be 20 to 40 % by weight. When the dental curable composition contains a polymerization initiator, the composition ratio may be 10 to 50 % by weight of the dental curable composition excluding the polymerization initiator and may be 20 to 40 % by weight. That is, when the dental curable composition does not contain a polymerization initiator, the composition ratio may be 10 to 50 parts by weight with respect to 100 parts by weight of the whole of the dental curable composition, and may be 20 to 40 parts by weight. When the dental curable composition contains a polymerization initiator, the composition ratio may be 10 to 50 parts by weight with respect to 100 parts by weight of the dental curable composition excluding the polymerization initiator, and may be 20 to 40 parts by weight. When the content is lower than 10 % by weight, there is a case where the mechanical strength of the cured product decreases. Furthermore, when the content exceeds 50 % by weight, there is a case where the viscosity of the paste is too high to decrease operability.

It is not necessary that the crushed type inorganic filler is manufactured or obtained as a single particle, and the crushed type inorganic filler may be obtained by mixing two or more crushed type inorganic fillers with different average particle diameters and/or composition ratios, as long as the average particle diameter and/or composition ratio of each falls within the above ranges.

It is also effective to use a crushed type inorganic filler surface-treated with silane coupling material represented by formula (1) and a crushed type inorganic filler surface-treated with a surface treatment agent other than the surface-treated with silane coupling material represented by formula (1) in combination.

By containing a crushed type inorganic filler, mechanical strength of the cured product is excellent and the shapability of the paste is superior. Furthermore, by combining the crushed type inorganic filler with the spherical nanofiller, it is possible to achieve the excellent flowability of the paste in the dental curable composition of the present invention. In the paste with high filling rate of the inorganic filler, for example, in the case of using a large amount of the crushed type inorganic filler, when the force is applied to the paste, because the crushed type inorganic fillers catch on each other and interfere each other, the viscosity of paste increases to impar flowability of the paste.

The dental curable composition of the present invention may contain, in addition to the inorganic filler surface-treated with silane coupling material represented by formula (1), an crushed type inorganic filler not surface-treated with silane coupling material represented by formula (1) as other filler described below. When the dental curable composition of the present invention does not contain the silane coupling material represented by formula (1) itself, the dental curable composition of the present invention may contain, in addition to the inorganic filler surface-treated with silane coupling material represented by formula (1), a crushed type inorganic filler not surface-treated with silane coupling material represented by formula (1) as other filler described below.

The shape of the inorganic filler is not particularly limited, but may be an aggregated type inorganic filler. When the inorganic filler is an aggregate, the average particle diameter described below means the average particle diameter of the aggregate. The aggregated type inorganic filler is a filler is granulated through a granulation process such as spray drying, from a nanoscale inorganic filler. By the cohesive force between the granulated fillers, it is expected to enhance the mechanical strength of the dental curable composition after curing.

The average particle diameter of the aggregated type inorganic filler used in the present invention may be 0.01 to 100 µm, and from the viewpoint of mechanical strength, may be 0.01 to 20 µm, or 0.01 to 10 µm. When the average particle diameter is less than 0.01 µm, there is a case where the specific surface area of the aggregated inorganic filler increases and the flowability of the paste decreases. On the other hand, when the average particle diameter exceeds 100 µm, the dispersibility of the aggregated type inorganic filler in the dental curable composition may deteriorate and it is impossible to manufacture a uniform paste.

The aggregated type inorganic filler used in the present invention may be an aggregated type inorganic filler hydrophobic-treated with a surface treatment agent. The hydrophobic treatment may be performed before surface treatment with silane coupling material represented by formula (1) and may be performed after surface treatment with silane coupling material represented by formula (1). Furthermore, the hydrophobic-treated aggregated type inorganic filler may be not surface-treated with the silane coupling material represented by formula (1). By containing a hydrophobic-treated aggregated type inorganic filler, it is possible to enhance shapability and improve the mechanical strength of the dental curable composition after curing.

The dental curable composition of the present invention may contain an aggregated type inorganic filler as the inorganic filler surface-treated with a silane coupling material represented by formula (1). The composition ratio of the aggregated type inorganic filler after surface-treatment with silane coupling material represented by formula (1) in the dental curable composition of the present invention is not particularly limited. However, when the dental curable composition does not contain a polymerization initiator, the composition ratio may be 10 to 60 % by weight of the whole of the dental curable composition, and may be 20 to 50 % by weight. When the dental curable composition contains a polymerization initiator, the composition ratio may be 10 to 60 % by weight of the dental curable composition excluding the polymerization initiator and may be 20 to 50 % by weight. That is, when the dental curable composition does not contain a polymerization initiator, the composition ratio may be 10 to 60 parts by weight with respect to 100 parts by weight of the whole of the dental curable composition, and may be 20 to 50 parts by weight. When the dental curable composition contains a polymerization initiator, the composition ratio may be 10 to 60 parts by weight with respect to 100 parts by weight of the dental curable composition excluding the polymerization initiator, and may be 20 to 50 parts by weight. When the content is lower than 10 % by weight, there is a case where the mechanical strength of the cured product decreases. Furthermore, when the content exceeds 60 % by weight, the viscosity of the prepared paste is too high to decrease operability.

The agglomerated inorganic filler in the present invention is a zirconium silicate consisting of "SiO₂/ZrO₂". The composition ratio is not particularly limited but may be "SiO₂/ZrO₂ = 50/50 to 99/1" and may be "SiO₂/ZrO₂ = 70/30 to 90/10". When the ratio of the ZrO₂ is less than 1, there is a case where the mechanical strength of the cured product decreases. Furthermore, when the ratio is more than 50, there is a case where the transparency of the dental curable composition is not be achieved.

When the dental curable composition contains the silane coupling material represented by formula (1) itself in the present invention, the composition ratio of the aggregated type inorganic filler that is not surface treated with the silane coupling material represented by formula (1) in the dental curable composition of the present invention is not particularly limited. However, when the dental curable composition does not contain a polymerization initiator, the composition ratio may be 9 to 55 % by weight of the whole of the dental curable composition, and may be 19 to 47 % by weight. When the dental curable composition contains a polymerization initiator, the composition ratio may be 9 to 55 % by weight of the dental curable composition excluding the polymerization initiator and may be 19 to 47 % by weight. That is, when the dental curable composition does not contain a polymerization initiator, the composition ratio may be 9 to 55 parts by weight with respect to 100 parts by weight of the whole of the dental curable composition, and may be 19 to 47 parts by weight. When the dental curable composition contains a polymerization initiator, the composition ratio may be 9 to 55 parts by weight with respect to 100 parts by weight of the dental curable composition excluding the polymerization initiator, and may be 19 to 47 parts by weight. When the content is lower than 9 % by weight, there is a case where the mechanical strength of the cured product decreases. Furthermore, when the content exceeds 55 % by weight, there is a case where the viscosity of the paste is too high to decrease operability.

It is not necessary that the aggregated type inorganic filler is manufactured or obtained as a single particle, and the aggregated type inorganic filler may be obtained by mixing two or more aggregated type inorganic fillers with different average particle diameters and/or composition ratios, as long as the average particle diameter and/or composition ratio of each falls within the above ranges.

It is also effective to use an aggregated type inorganic filler surface-treated with silane coupling material represented by formula (1) and an aggregated type inorganic filler surface-treated with a surface treatment agent other than the surface-treated with silane coupling material represented by formula (1) in combination.

By containing an aggregated type inorganic filler, mechanical strength of the cured product is excellent and the shapability of the paste is superior. Furthermore, by combining the aggregated type inorganic filler with the spherical nanofiller, it is possible to achieve the excellent flowability of the paste in the dental curable composition of the present invention. In the paste with high filling rate of the inorganic filler, for example, in the case of using a large amount of the aggregated type inorganic filler, when the force is applied to the paste, because the aggregated type inorganic fillers catch on each other and interfere each other, the viscosity of paste increases to impar flowability of the paste.

The dental curable composition of the present invention may contain, in addition to the inorganic filler surface-treated with silane coupling material represented by formula (1), an agglomerated inorganic filler not surface-treated with silane coupling material represented by formula (1) as other filler described below. When the dental curable composition of the present invention does not contain the silane coupling material represented by formula (1) itself, the dental curable composition of the present invention may contain, in addition to the inorganic filler surface-treated with silane coupling material represented by formula (1), an agglomerated inorganic filler not surface-treated with silane coupling material represented by formula (1) as other filler described below.

### [Other filler]

The dental curable composition of the present invention may contain various known fillers other than the inorganic filler surface-treated with silane coupling material represented by formula (1) as other filler, if necessary. Examples of the other filler used in the present invention include the above described inorganic filler not surface-treated, the above described inorganic filler not surface-treated using a silane coupling material represented by the chemical structural formula of formula (1), the above described inorganic filler surface-treated using a surface treatment agent other than the silane coupling material represented by formula (1), organic fillers and organic-inorganic composite fillers.

When the dental curable composition contains the silane coupling material represented by formula (1) itself, it is possible to compound other filler into the dental curable composition after the surface treatment with the silane coupling material represented by formula (1) is completed.

The inorganic filler surface-treated with a surface treatment agent other than the silane coupling material represented by formula (1) may be an inorganic filler surface-treated with only the surface treatment agent other than the silane coupling material represented by formula (1). Examples of such surface treatment include surface treatment with 3-metacryloxypropyl trimethoxysilane, surface treatment with 8-metacryloxyoctyl trimethoxysilane and surface treatment with titanium acetylacetonate. By using two or more types of surface treatment agents including the silane coupling material represented by formula (1) and a surface treatment agent other than the silane coupling material represented by formula (1), it becomes possible to freely adjust properties such as the polymerizability of the polymerizable monomer, the dispersibility of the filler, the paste operability and shapability, the stability of the paste and the mechanical strength after curing in the whole of dental curable composition.

When the dental curable composition does not contain a polymerization initiator, the content of the inorganic filler surface-treated with surface treatment agent other than the silane coupling material represented by formula (1) may be 1 to 50 % by weight with respect to the whole of the dental curable composition, and may be 10 to 40 by weight. That is, the content may be 1 to 50 parts by weight with respect to 100 parts by weight of the whole of the dental curable composition, and may be 10 to 40 parts by weight. When the content is less than 1 % by weight, there is a possibility that the operability of the paste decreases and when the content exceeds 50 parts by weight, there is a possibility that the mechanical strength after curing decreases. Furthermore, when the dental curable composition contains a polymerization initiator, the content may be 20 to 40 % by weight of the dental curable composition excluding the polymerization initiator. That is, the content may be 20 to 40 parts by weight with respect to 100 parts by weight of the dental curable composition excluding the polymerization initiator. When the content is less than 20 % by weight, there is a possibility that the operability of the paste decreases, and when the content exceeds 40 % by weight, there is a possibility that the mechanical strength after curing decreases.

Examples of the organic filler include elastomers such as polyvinyl acetate, polyvinyl alcohol and a styrene-butadiene rubber, non-crosslinkable (meth)acrylate polymers each being a homopolymer of a monofunctional (meth)acrylate polymerizable monomer, such as polymethyl methacrylate (PMMA), polyethyl methacrylate, polypropyl methacrylate and polybutyl methacrylate, crosslinkable (meth)acrylate polymers obtained by copolymerizing a monofunctional (meth)acrylate polymerizable monomer with a polymerizable monomer having two or more functional groups, and polyvinyl acetate, polyethylene glycol, polypropylene glycol and polyvinyl alcohol, but are not limited thereto.

In addition, examples of the organic/inorganic composite filler include one obtained by covering the surface of a filler with a polymerizable monomer by polymerization, one obtained by mixing a filler and a polymerization monomer and polymerizing the monomer, and thereafter grinding the resultant to a proper particle size, or one obtained by dispersing a filler in a polymerizable monomer in advance for emulsion polymerization or suspension polymerization, but are not limited thereto at all.

These of the organic filler and the organic-inorganic composite filler may be surface-treated or may be not surface-treated.

The dental curable composition of the present invention may not contain an inorganic filler that is not surface-treated using the silane coupling material represented by formula (1). The dental curable composition of the present invention may not contain a filler other than the inorganic filler surface-treated with silane coupling material represented by formula (1). The dental curable composition of the present invention may not contain an inorganic filler that is not surface-treated. The dental curable composition of the present invention may not contain a filler that is not surface-treated.

### [Radical polymerizable monomer]

When the dental curable composition does not contain a polymerization initiator, the content of the radical polymerizable monomer compounded in the specific dental curable composition containing the inorganic fillers of the present invention may be 10 to 60 % by weight of the whole of the dental curable composition, and may be 15 to 30 % by weight. When the dental curable composition contains a polymerization initiator, the content may be 10 to 60 % by weight of the dental curable composition excluding the polymerization initiator and may be 15 to 30 % by weight. That is, when the dental curable composition does not contain a polymerization initiator, the content may be 10 to 60 parts by weight with respect to 100 parts by weight of the whole of the dental curable composition, and 15 to 30 parts by weight. When the dental curable composition contains a polymerization initiator, the content may be 10 to 60 parts by weight with respect to 100 parts by weight of the dental curable composition excluding the polymerization initiator, and 15 to 30 parts by weight. When the content is lower than 10 % by weight, there is a case where the viscosity of the dental curable composition is too high to impair the operability of the paste. Furthermore, when the content exceeds 60 % by weight, there is a case where the mechanical strength of the cured product of the dental curable composition decreases.

As the radical polymerizable monomer used in the dental curable composition of the present invention, the radical polymerizable monomer used in the dental field can be used without limitation, but may have an urethane bond or a hydrogen bond in their molecular backbone. This is to effectively form a hydrogen bond with the silane coupling material used in the present invention. Examples of a monomer not containing an urethane bond or a hydrogen bond include phenoxyethyl acrylate, methyl methacrylate, tetrahydrofurfuryl acrylate, triethyleneglycol diacrylate, trimethylolpropane triacrylate, dipentaerythritol hexaacrylate and bisphenol A diethylethoxylate diacrylate.

The number of functional group of the radical polymerizable monomer used in the present invention may be 2 to 9 functionalities. In the case of mono functionality, there is a case where a crosslinked structure is not formed and sufficient mechanical strength is not imparted to the dental curable composition. On the other hand, in the case of 10 or more functionalities, there is a case where the density of the crosslinked structure becomes too high to cause the dental curable composition to become brittle. In the present invention, the functionality of a radical polymerizable monomer means the number of polymerizable groups, such as (meth)acryloyl groups.

The radical polymerizable monomer may have a urethane bond. For the specific compounding amount of the radical polymerizable monomer containing a urethane bond, when the dental curable composition does not contain a polymerization initiator, the content may be 10 to 50 % by weight of the whole of the dental curable composition, and may be 15 to 30 % by weight. When the dental curable composition contains a polymerization initiator, the content may be 10 to 50 % by weight of the dental curable composition excluding the polymerization initiator and may be 15 to 30 % by weight. That is, when the dental curable composition does not contain a polymerization initiator, the content may be 10 to 50 parts by weight with respect to 100 parts by weight of the whole of the dental curable composition, and may be 15 to 30 parts by weight. When the dental curable composition contains a polymerization initiator, the content may be 15 to 30 parts by weight with respect to 100 parts by weight of the dental curable composition excluding the polymerization initiator, and may be 10 to 50 parts by weight. When the content is lower than 10 % by weight, there is a case where the mechanical strength of the cured product of the dental curable composition decreases. When the content exceeds 50 % by weight, there is a case where the viscosity of the the dental curable composition is too high to impair the operability of the paste. In the present invention, a urethane bond means a bond having -NH-C(=O)-O- group. Examples of such radical polymerizable monomers include di(meth)acrylates having a bifunctionality or more and urethane bond, which are derived from an adduct of a polymerizable monomer having a hydroxy group such as 2-hydroxyethyl (meth)acrylate, 2-hydroxypropyl (meth)acrylate and 3-chloro-2-hydroxypropyl (meth)acrylate, and a diisocyanate compound such as methylcyclohexane diisocyanate, methylene bis (4-cyclohexyl isocyanate), hexamethylene diisocyanate, trimethyl hexamethylene diisocyanate, isophorone diisocyanate, diisocyanate methylbenzene and 4,4-diphenylmethane diisocyanate.

The radical polymerizable monomer may have a hydrogen bond. For the specific compounding amount of the radical polymerizable monomer containing a hydrogen bond, when the dental curable composition does not contain a polymerization initiator, the content may be 1 to 20 % by weight of the whole of the dental curable composition, and may be 1 to 10 % by weight. When the dental curable composition contains a polymerization initiator, the content may be 1 to 20 % by weight of the dental curable composition excluding the polymerization initiator and may be 1 to 10 % by weight. That is, when the dental curable composition does not contain a polymerization initiator, the content may be 1 to 20 parts by weight with respect to 100 parts by weight of the whole of the dental curable composition, and may be 1 to 10 parts by weight. When the dental curable composition contains a polymerization initiator, the content may be 1 to 20 parts by weight with respect to 100 parts by weight of the dental curable composition excluding the polymerization initiator, and may be 1 to 10 parts by weight. When the content is lower than 1 % by weight, there is a case where the viscosity of the the dental curable composition is too high to impair the operability of the paste. When the content exceeds 20 % by weight, there is a case where the mechanical strength of the cured product of the dental curable composition decreases. Examples of such radical polymerizable monomers include 2-hydroxy-1,3-dimethacryloxy propane, 4-hydroxybutyl acrylate, 2-hydroxyethyl acrylate, 2-hydroxypropyl acrylate, 2-hydroxyethyl methacrylate, 2-hydroxypropyl methacrylate, 2-hydroxy-3-phenoxypropyl acrylate, 2-acryloyloxyethyl-2-hydroxyethyl phthalate and 2-acryloyloxyethyl hexahydrophthalate.

The radical-polymerizable monomer may be a monomer having one or more hydroxyl groups and two or more polymerizable functional groups. In the present invention, two or more polymerizable functional groups means the number of the polymerizable group, such as two or more (meth)acryloyl groups. Examples of such radical polymerizable monomer include 2-hydroxy-1,3-dimethacryloxy propane, 2-hydroxy-1,3-diacryloxy propane, 2-hydroxy-3-acryloxypropyl methacrylate, 2-acryloxyethyl phthalate and acryloxyethyl monomethyl tetrahydrophthalate. By containing one or more hydroxyl groups and two or more polymerizable functional groups, it is possible to achieve appropriate paste dispersibility to achieve high mechanical strength after curing.

The radical polymerizable monomer having one or more hydroxyl groups and two or more polymerizable functional groups may be a monomer represented by formula (2). For the monomer represented by formula (2), when the dental curable composition does not contain a polymerization initiator, the content may be 1 to 20 % by weight of the whole of the dental curable composition, and may be 1 to 10 % by weight. When the dental curable composition contains a polymerization initiator, the content may be 1 to 20 % by weight of the dental curable composition excluding the polymerization initiator and may be 1 to 10 % by weight. That is, when the dental curable composition does not contain a polymerization initiator, the content may be 1 to 20 parts by weight with respect to 100 parts by weight of the whole of the dental curable composition, and may be 1 to 10 parts by weight. When the dental curable composition contains a polymerization initiator, the content may be 1 to 20 parts by weight with respect to 100 parts by weight of the dental curable composition excluding the polymerization initiator, and may be 1 to 10 parts by weight. When the content is lower than 1 % by weight, there is a case where the viscosity of the the dental curable composition is too high to impair the operability of the paste. When the content exceeds 20 % by weight, there is a case where the mechanical strength of the cured product of the dental curable composition decreases. By optimal content, a hydrogen bond is formed with an amide bondin in the silane coupling material to improve the mechanical strength of the cured body.

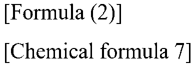

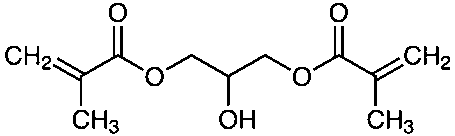

When the dental curable composition of the present invention contains the silane coupling material represented by formula (1) itself, the weight ratio of the silane coupling material represented by formula (1) to the radical polymerizable monomer having a compound represented by formula (2) may be in a range of 1:0.5 to 1:20. When the weight ratio of the radical polymerizable monomer having a compound represented by formula (2) to the silane coupling material represented by formula (1) is less than 1:0.5, there is a possiblity that the viscosity of the dental curable composition becomes excessively high to impaire the operability of the paste. When the weight ratio exceeds 1:20, there is a possiblity that the mechanical strength of the dental curable composition decreases.

### [Polymerization initiator]

The polymerization initiator used in the present invention is not particularly limited, and any known radical generators may be used without any limitations. Generally, the polymerization initiators are roughly classified into those in which polymerization is initiated by heating (thermal polymerization initiator), those in which polymerization is initiated by light irradiation (photo polymerization initiator) and those in which polymerization is initiated by mixing two forms of composition (chemical polymerization initiator). From the viewpoint of mechanical strength after curing, thermal polymerization initiators are preferably used as the polymerization initiator in the present invention.

As the thermal polymerization initiator, an organic peroxide can be preferably used, and specific examples include benzoyl peroxide, parachlorobenzoyl peroxide, 1,1-bis (tert-butylperoxy) cyclohexane, 2,4-dichlorobenzoyl peroxide, acetyl peroxide, lauroyl peroxide, tert-butyl peroxide, cumene hydroperoxide, 2,5-dimethylhexane, 2,5-dihydroperoxide, methyl ethyl ketone peroxide, and tert-butyl peroxybenzoate.

The thermal polymerization initiator in the present invention may be selected from thermal polymerization initiators commonly used in the general industrial field, for example, those having a specific ten hour half-life temperature, to use.

The ten hour half-life temperature of the thermal polymerization initiator in the present invention is 50 to 200 °C, and may be 90 to 170 °C from the viewpoint of the mechanical strength. By setting the 10-hour half-life temperature within an appropriate range, it is possible to improve the polymerization rate to improve the mechanical strength of the cured product even in the case of using a radical polymerizable monomer having high-viscosity. When the ten hour half-life temperature is below 50 °C, there is a risk that sufficient polymerization rate may not be achieved in the case of using a polymerizable monomer having high-viscosity and therefore the mechanical strength decreases. When the ten hour half-life temperature is higher than 200 °C, there is a risk that a decrease in the mechanical strength of the cured product and yellowing are caused by thermal degradation. The ten hour half-life temperature in the present invention refers to the temperature at which the half-life of the thermal polymerization initiator is 10 hours. The half-life refers to the time required for the concentration of the thermal polymerization initiator to decrease to half of its initial value. Examples of the thermally polymerizable initiators havnig a ten hour half-life temperature between 70 °C and 170 °C include 1,1-bis (tert -butylperoxy) cyclohexane, 2,2-bis [4,4-bis (isobutylperoxy) cyclohexyl] propane, tert-butylperoxy isopropylcarbonate, tert-butyldodecanoate peroxy, tert-butyl carbonate (2-ethylhexyl), bis (1-phenyl-1-methylethyl) peroxide, di-tert-butyl peroxide and 2,5-dimethyl-2,5-di-(t-butylperoxy)-hexane-3.

Specific examples of the photo polymerization initiator include α-diketones such as camphorquinone, diphenyl (2,4,6-trimethylbenzoyl) phosphine oxide, phenyl bis (2,4,6-trimethylbenzoyl) phosphine oxide, acetonaphthene, p,p'-dimethoxybenzyl, p,p'-dichlorobenzylacyl, pentanedione, 1,2-phenanthroquinone, 1,4-phenanthroquinone, 3,4-phenanthroquinone, 9,10-phenanthroquinone and naphthoquinone, benzoin, benzoin alkyl ethers such as benzoin methyl ether and benzoin ethyl ether, thioxanthone, 2-chlorothioxanthone, 2-methylthioxanthone, 2-isopropylthioxanthone, 2-methoxithioxanthone, 2-hydroxithioxanthone and 2,4-diethylthioxanthone.

Examples of a chemical polymerization initiators which can be used in that include a redox-type polymerization initiator system comprising organic peroxide/amine compound, organic peroxide/amine compound/sulfinate, organic peroxide/amine compound/borate compound, and a polymerization catalyst system such as organic boron compounds, perborates, permanganates, and persulfates which initiate polymerization by reacting with oxygen or water. Further, sulfinates, borate compounds and barbituric acids can initiate polymerization in the presence of water or a polymerizable monomer having an acidic group.

Examples of the organic peroxide are not limited to, but include benzoyl peroxide, parachlorobenzoyl peroxide, 2, 4-dichlorobenzoyl peroxide, acetyl peroxide, lauroyl peroxide, tertiary butyl peroxide, cumene hydroperoxide, 2,5-dimethylhexane, 2, 2,5-dihydroperoxide, methyl ethyl ketone peroxide, and tertiary butyl peroxybenzoate. The organic peroxides can be used alone, or in a combination of a few of them.

As the amine compound, secondary or tertiary amine in which an amine group is bound to an aryl group is preferable, and examples are not limited to, but include N,N-dimethyl-p-toluidine, N,N, dimethylaniline, N-[β]-hydroxyethyl-aniline, N,N-di([B]-hydroxyethyl)-aniline, N,N-di([beta]-hydroxyethyl)-p-toluidine, N-methyl-aniline, and N-methyl-p-toluidine. The amine compounds may be used alone, or in combination of a few of them.

Examples of sulfinates are not limited to, but include sodium benzenesulfinate, lithium benzenesulfinate, and sodium p-toluenesulfinate. The sulfuric acid salts may be used alone, or in combination of a few of them.

Examples of the borate compound are not limited to, but include a sodium salt, a lithium salt, a potassium salt, a magnesium salt, a tetrabutylammonium salt, and a tetramethylammonium salt of trialkylphenylboron, and trialkyl(p-fluorophenyl)boron (wherein an alkyl group is a n-butyl group, a n-octyl group, a n-dodecyl group etc.). The borate compounds may be used alone, or in a combination of few of them. Specific examples of the barbituric acids include, but are not limited to, barbituric acid, 1,3-dimethylbarbituric acid, 1,3-diphenylbarbituric acid, 1,5-dimethylbarbituric acid, 5-butylbarbituric acid, 5-ethylbarbituric acid, 5-isopropylbarbituric acid, 5-cyclohexylbarbituric acid, 1,3,5-trimethylbarbituric acid, 1,3-dimethyl-5-ethylbarbituric acid, 1,3-dimethyl-n-butylbarbituric acid, 1,3-dimethyl-5-isobutylbarbituric acid, 1,3-dimethyl-barbituric acid, 1,3-dimethyl-5-cyclopentylbarbituric acid, 1,3-dimethyl-5-cyclohexylbarbituric acid, 1,3-dimethyl-5-phenylbarbituric acid, 1-cyclohexyl-5-ethylbarbituric acid, 1-benzyl-5-phenylbarbituric acid and thiobarbituric acids, and salts thereof (particularly alkali metal or alkali earth metals). Examples of the salts thereof include, but are not limited to, sodium 5-butylbarbiturate, sodium 1,3,5-trimethylbarbiturate, calcium 1,3,5-trimethylbarbiturate and sodium 1-cyclohexyl-5-ethylbarbiturate. These barbiturates can also be used alone or in combination.

Among these chemical polymerization initiators, it is possible to use a combination of organic peroxide/tertiary amine.

These polymerization initiators can be used not only singly but also in combinations of two or more, regardless of the polymerization manner or the polymerization method. In addition, these polymerization initiators have no problem even if subjected to a secondary treatment such as encapsulation in a microcapsule, if necessary. The dental curable composition of the present invention may contain only thermopolymerization initiators as the polymerization initiator. The dental curable composition of the present invention may contain only photo polymerization initiators as the polymerization initiator.

The content of the polymerization initiator used in the present invention may be appropriately selected according to the intended use, and may be within the range of 0.01 to 10 parts by weight with respect to 100 parts by weight of the radical polymerizable monomer, and may be within the range of 0.1 to 5 parts by weight. When the amount of polymerization initiator is too much, there is a case where crack easily occurs in manufacturing a dental resin cured body, making production difficult. When the amount of polymerization initiator is too low, sufficient effect cannot be obtained and mechanical strength decreases.

In the dental curable composition of the present invention, known various additives can be mixed, if necessary. Examples of such additives which can be used in the present invention include a polymerization inhibitor, a chain transfer agent, a coloring material, a discoloration preventing material, a fluorescent material, an ultraviolet absorber and an antibacterial material. The dental curable composition of the present invention may contain only one or more selected from a polymerization inhibitor, a chain transfer agent, a colorant, a discoloration preventing agent, a fluorescent agent, an ultraviolet absorber and an antibacterial agent as the additives.

In the dental curable composition of the present invention, the cured body of the dental curable composition may have a bending strength of 300 MPa or more after storage in water at 37 °C for one week. By having a bending strength of 300 MPa or more, it is possible to provide a dental curable composition which is excellent in machinability into complex shapes and durability. The bending strength can be measured using a universal testing machine (manufactured by Instron) at a crosshead speed of 1 mm/min in accordance with ISO 6872.

In the dental curable composition of the present invention, the cured body of the dental curable composition may have a flexural modulus of 20 GPa or more after storage in water at 37 °C for one week. By having a flexural modulus of 20 GPa or more, it is possible to provide a dental curable composition which is excellent in machinability into complex shapes and durability. The flexural modulus can be measured using a universal testing machine (manufactured by Instron) at a crosshead speed of 1 mm/min in accordance with ISO 6872.

The dental curable composition of the present invention can be used, for example, in manufacture of a dental resin cured body for cutting, in manufacture of a dental composite resin, and as a dental curable composition for 3D printer.

### [Examples]

Hereinafter, example of the present invention are specifically described. However, the present invention is not intended to be limited to these Examples.

### [Inorganic filler]

ZSF: aggregated type zirconium silicate (d₅₀: 2 µm)
GM32087: crushed type strontium alumoborosilicate glass (d₅₀: 0.4 µm, manufactured by SCHOTT)
Aerosil R-7200: irregular non crystalline silica (primary particle diameter: 12 nm, manufactured by Japan Aerosil)
Aerosil OX-50: irregular non crystalline silica (primary particle diameter: 40 nm, manufactured by Japan Aerosil)
Admanano YC100C-SM2: spherical non crystalline silica (primary particle diameter: 100 nm, manufactured by Admatex)

### [Surface treatment agent]

### <Silane coupling material represented by formula (1)>

X-12-1370: N-(3-trimethoxysilyl) propyl) methacrylamide (molecular weight 247, Shin-Etsu Chemical Co., Ltd.)

### <Silane coupling material other than silane coupling material represented by formula (1)>

KBM-503: 3-methacryloxypropyl trimethoxysilane (molecular weight 248, manufactured by Shin-Etsu Chemical Co., Ltd.)
KBM-502: 3-methacryloxypropyl methyl dimethoxysilane (molecular weight 232, manufactured by Shin-Etsu Chemical Co., Ltd.)
KBM-5803: 8-methacryloxyoctyl trimethoxysilane (molecular weight 319, manufactured by Shin-Etsu Chemical Co., Ltd.)
TC-100: titanium acetylacetonate (manufactured by Matsumoto Fine Chemical Co., Ltd.)
TC-401: titanium tetraacetylacetonate (manufactured by Matsumoto Fine Chemical Co., Ltd.)
TC-750: titanium ethyl acetoacetate (manufactured by Matsumoto Fine Chemical Co., Ltd.)
ZC-200: zirconium octanoate compound (manufactured by Matsumoto Fine Chemical Co., Ltd.)
ZC-580: zirconium ethyl acetoacetate (manufactured by Matsumoto Fine Chemical Co., Ltd.)
KBE-9103P: 3-[(1,3-dimethylbutylidene) amino] propyltriethoxysilane (molecular weight 304, manufactured by Shin-Etsu Chemical Co., Ltd.)
X-12-1135: organosiloxane containing carboxyl group (manufactured by Shin-Etsu Chemical Co., Ltd.)

### [Radical-polymerizable monomer]

### <Radical-polymerizable monomer having urethane bond>

GENOMER 4267: aliphatic urethane acrylate (viscosity 16,000 mPa·s at 60 °C, manufactured by RAHN AG)
GENOMER 4247: aliphatic urethane methacrylate (viscosity 10,000 mPa·s at 25 °C, manufactured by RAHN AG)
GENOMER 4425: aliphatic urethane acrylate (viscosity 4,500 mPa·s at 25 °C, manufactured by RAHN AG)

### <Radical-polymerizable monomer having hydrogen bond>

HEMA: 2-hydroxyethyl methacrylate (viscosity: 7 mPa·s (20 °C), manufactured by Mitsubishi Chemical)
M-600A: 2-hydroxy-3-phenoxypropyl acrylate (viscosity: 150 mPa·s (25 °C), manufactured by Kyoei Chemical)

### < Polymerizable monomer having one or more hydroxyl group and two or more polymerizable functional groups >

G-201P: 2-hydroxy-3-acryloyloxypropyl methacrylate (viscosity: 50 mPa·s, manufactured by Kyoei Chemical Co., Ltd.)

### <Polymerizable monomer represented by Formula (2) >

NK ester 701: 2-hydroxy-1,3-dimethacryloxypropane (viscosity: 37 mPa·s (25 °C), manufactured by Shin Nakamura Chemical Co., Ltd.)

### <Other radical polymerizable monomer>

14EG: polyethylene glycol methacrylate (viscosity: 60 mPa·s (25 °C), manufactured by Kyoeisha Chemical Co., Ltd.)
BP-2EM: EO adduct diacrylate of bisphenol A (viscosity: 1400 mPa·s (25 °C), manufactured by Kyoeisha Chemical Co., Ltd.)
3G: triethylene glycol dimethacrylate (viscosity: 9 mPa·s (25 °C), manufactured by Shin Nakamura Chemical Co., Ltd.)

### [Polymerization initiator]

### <Thermalpolymerization initiator>

Perhexa C: 1,1-bis (tert-butylperoxy) cyclohexane (ten hour half-life temperature: 90.7°C, manufactured by NOF Corporation)
Perhexa V: n-Butyl = 4,4-bis (tert-butylperoxy) valerate (ten hour half-life temperature: 104.5°C, manufactured by NOF Corporation)
Perhexyl D: di-t-hexyl peroxide (ten hour half-life temperature: 116.4°C, manufactured by NOF Corporation)

### [Others]

γ-terpinene: γ-terpinene (molecular weight: 136.4, manufactured by Tokyo Chemical Industry Co., Ltd.)

### (Preparation of surface-treated inorganic filler)

Surface treatment with surface treatment agent was performed on 100 g of untreated inorganic filler in the combinations shown in Table 1. Specifically, a surface treatment solution (prepared by pre-stirring the surface treatment agent at the compounding amount described in Table 1, 6 g of water, 6 g of ethanol and 0.1 g of phosphoric acid) was added to 100 g of untreated inorganic filler and stirred and mixed for 15 minutes. Thereafter, heat treatment was performed at 115 °C for 3 hours to obtain a heat treated product.

### Treatment amount of each inorganic filler

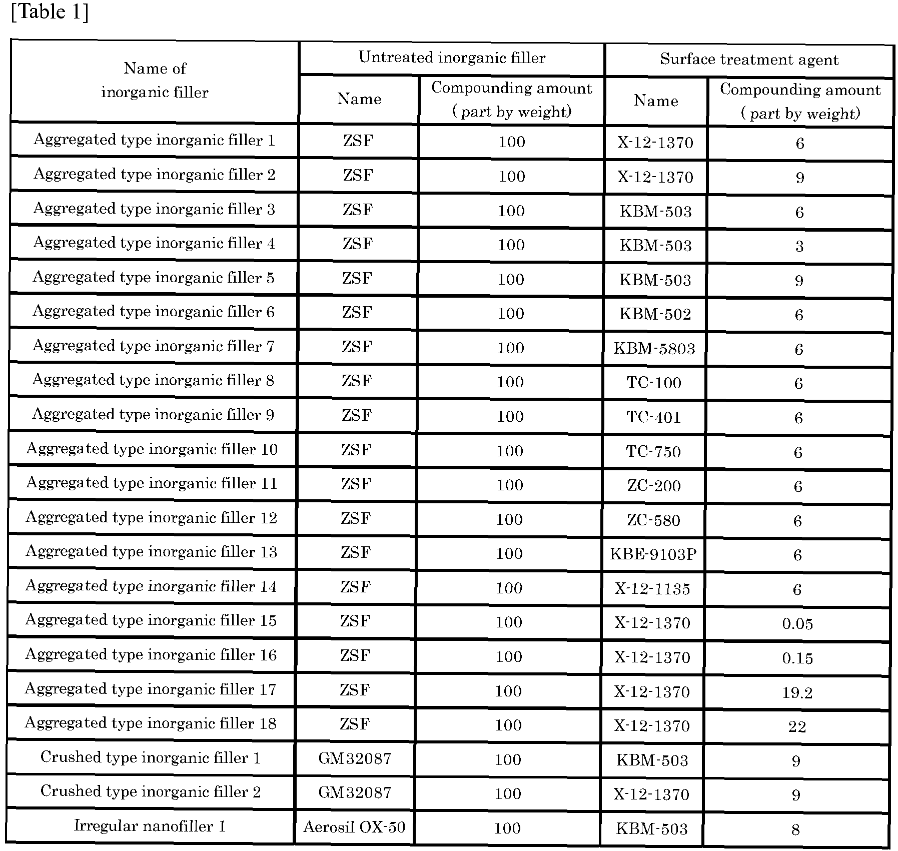

### (Preparation of dental resin cured body for cutting)

The compounding amount of each component in each Example and Comparative Example are shown in Tables 2 and 3. The components shown in Tables 2 and 3 were kneaded and defoamed under reduced pressure to prepare a mixture (dental curable composition). After filling the mixture into a die made by aluminum, the mixture was sandwiched between aluminum plates and performed with a vacuum pressure testing. Thereafter, the mixtures was cured by conducting hot press under the condition of 130 °C-3.5t-50min. Then, an additional heat treatment was performed for 8 hours at 100 °C to prepare a dental resin cured body for cutting used for the mechanical strength test.

### (Measurement method of mechanical strength of dental resin cured body for cutting)

A test piece (19.0 mm × 4.0 mm × 1.2 mm) was cut out from the dental resin cured body for cutting and was used as a test specimen after adjusting the surface with water-resistant polishing paper (# 2000). The prepared test piece was immersed in deionized water and stored at 37 °C for 7 days. Thereafter, the bending strength and flexural modulus were measured by using an Instron universal tester (Instron 5967 manufactured by Instron) at a crosshead speed of 1mm/min according to ISO 6872.

The test results of the prepared dental curable compositions are shown in Tables 2 to 7.

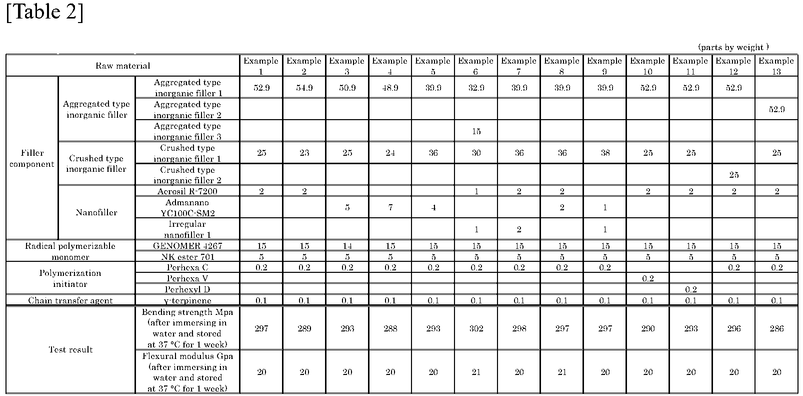

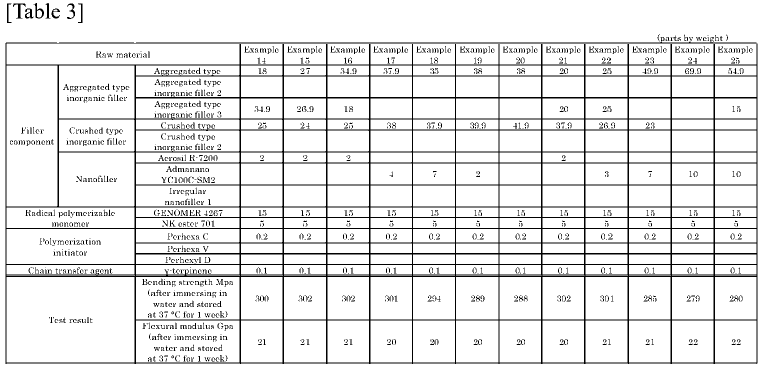

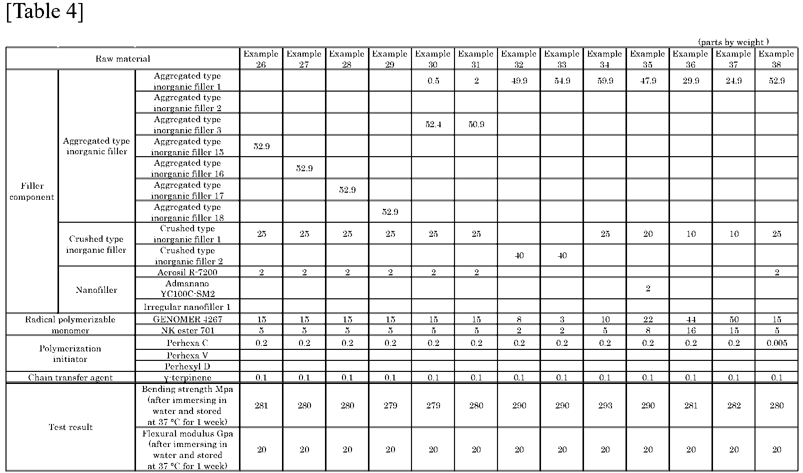

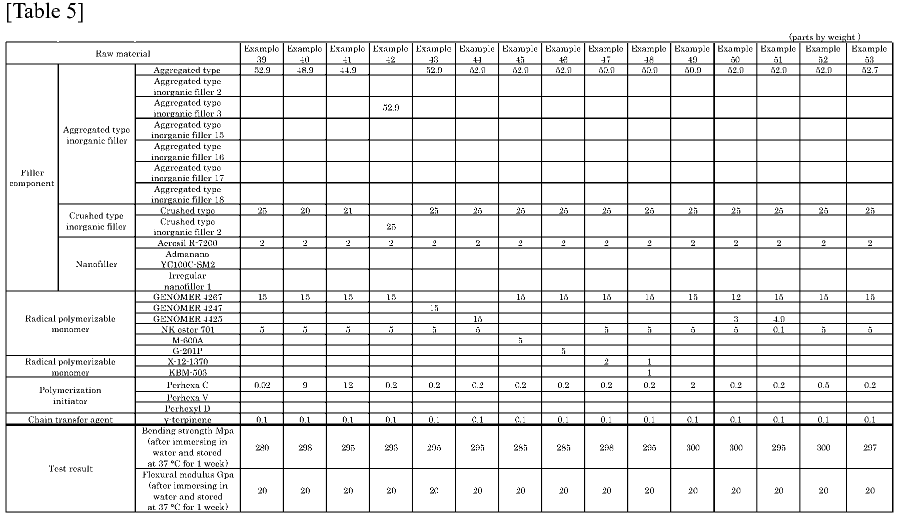

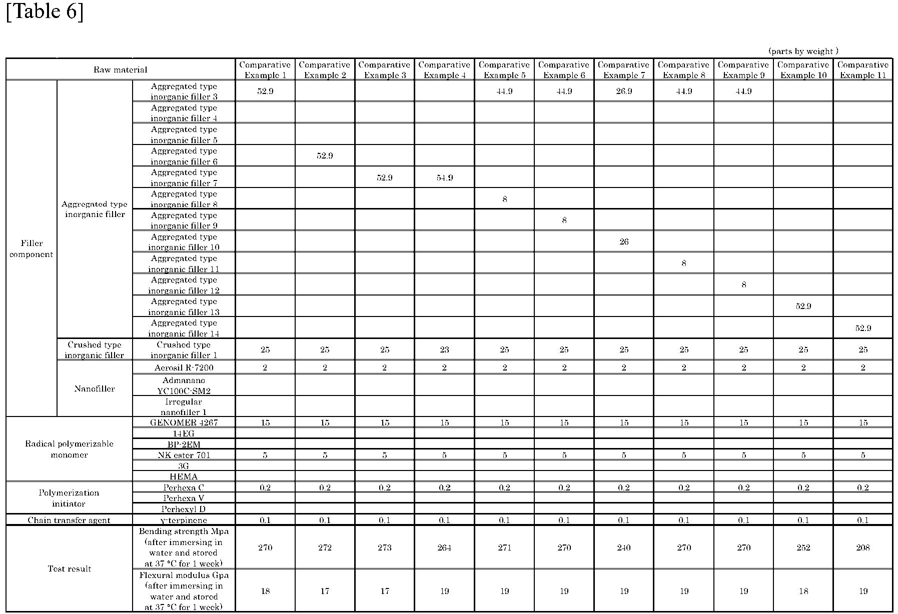

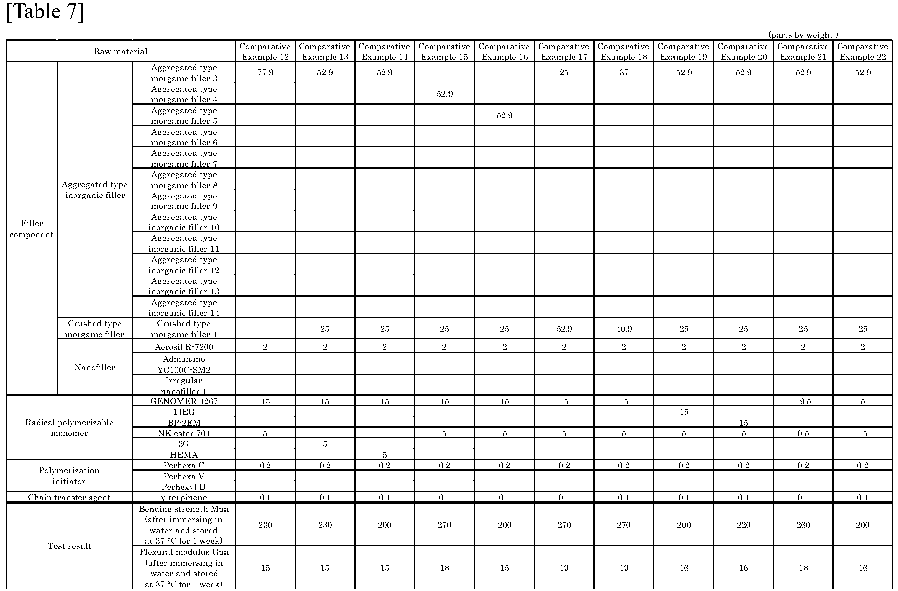

### [Examples 1-52]

It was confirmed that the dental curable compositions of Examples 1-52 exhibited high bending strength and flexural modulus and therefore exhibited excellent mechanical strength.

### [Comparative Examples 1-22]

It was confirmed in the dental curable compositions of Comparative Examples 1-22 that the mechanical strength decreased, because of not compounding the inorganic fillers surface-treated with X-12-1370.

Thus, the dental curable compositions prepared according to the present invention exhibited significantly higher bending strength compared to dental curable compositions (Comparative Examples 1 to 22) using conventionally used silane coupling materials (for example, KBM-503). The present invention enables to provide a dental curable composition having high mechanical strength after curing, which was unattainable with prior art.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this invention without difficulty. Accordingly, any such changes and modifications are intended to be included in the scope of the embodiments.

### [Industrial applicability]

According to the present invention, it is possible to provide a dental curable composition that can achieve high mechanical strength after curing.

## Claims

1. A dental curable composition comprising a silane coupling material represented by formula (1) and/or an inorganic filler surface-treated with silane coupling material represented by formula (1).

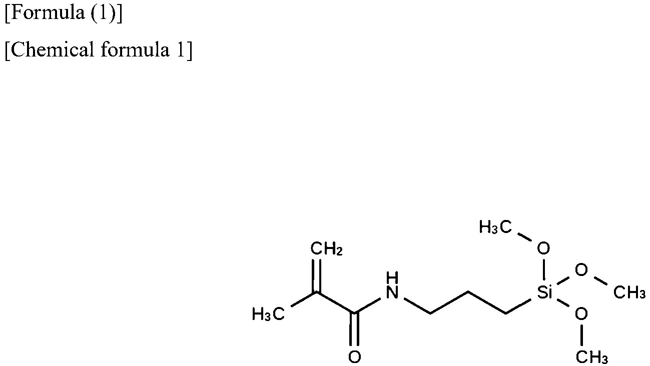

2. The dental curable composition according to claim 1, comprising the inorganic filler surface-treated with silane coupling material represented by formula (1).

3. The dental curable composition according to claim 2, wherein
a treatment amount of the silane coupling material in the surface treatment is 0.1 to 20 parts by weight with respect to 100 parts by weight of the inorganic filler.

4. The dental curable composition according to claim 2, wherein
a compounding amount of the inorganic filler surface-treated with silane coupling material represented by formula (1) is 1 to 90 parts by weight with respect to 100 parts by weight of the whole of the dental curable composition.

5. The dental curable composition according to any one of claims 1 to 4, further comprising
a radical polymerizable monomer, and
a polymerization initiator.

6. The dental curable composition according to claim 5, further comprising
an inorganic filler surface-treated with a surface treatment agent other than the silane coupling material represented by formula (1).

7. The dental curable composition according to claim 5, wherein
the radical polymerizable monomer has a urethane bond.

8. The dental curable composition according to claim 5, wherein
the radical polymerizable monomer has one or more hydroxyl groups and two or more polymerizable functional groups.

9. The dental curable composition according to claim 8, wherein
the radical polymerizable monomer includes a monomer represented by formula (2).

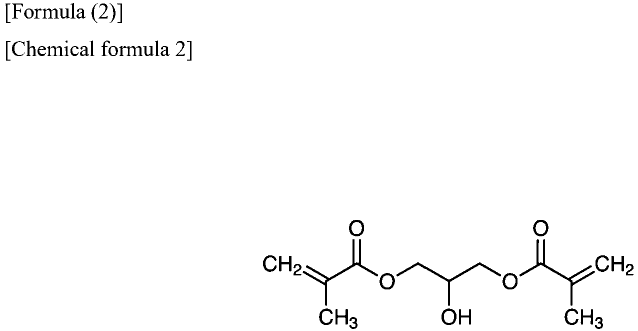

10. The dental curable composition according to claim 9, wherein
a compounding amount of the radical polymerizable monomer containing the compound represented by formula (2) is 1 to 10 parts by weight with respect to 100 parts by weight of the whole of the dental curable composition.

11. The dental curable composition according to claim 9, wherein
a weight ratio of the silane coupling material represented by formula (1) to the radical polymerizable monomer including the compound represented by formula (2) is in a range of 1:0.5 to 1:20.

12. The dental curable composition according to claim 2, wherein
the inorganic filler surface-treated with silane coupling material represented by formula (1) is one or more selected from a group consisting of a spherical nanofiller, a crushed type inorganic filler and an aggregated type inorganic filler and has an average particle diameter of 0.01 to 1 µm.

13. The dental curable composition according to claim 12, comprising
the aggregated type inorganic filler consists of SiO₂: 50-99 % by weight and ZrO₂: 1-50 % by weight.

14. The dental curable composition according to claim 5, wherein
a ten hour half-life temperature of the polymerization initiator is between 70 °C and 170 °C.

15. The dental curable composition according to claim 5, wherein
a cured body of the dental curable composition has a bending strength of 300 MPa or more after storage in water at 37 °C for one week.

16. The dental curable composition according to claim 5, wherein
a cured body of the dental curable composition has a flexural modulus of 20 GPa or more after storage in water at 37 °C for one week.

17. The dental curable composition according to claim 5, wherein
the dental curable composition is used in one or more selected from a group consisting of manufacture of a dental resin cured body for cutting, manufacture of a dental composite resin and manufacture of a dental curable composition for 3D printer.

18. An inorganic filler surface-treated with silane coupling material represented by formula (1).
